# EUROPEAN PATENT APPLICATION

(11) **EP 4 190 915 A1**
(43) Date of publication of application: **07.06.2023**
(21) Application number: 21849165.2
(22) Date of filing: 08.04.2021
(51) Int. Cl.: C12Q 1/6883, A61K 31/7088, A61P 1/16, A61P 3/00

(54) **BIOMARKER FOR DIAGNOSING NONALCOHOLIC STEATOHEPATITIS USING MICRORNA COMBINATION**

(30) Priority: 28.07.2020 KR 20200093534
(71) Applicant: Korea University Research and Business Foundation, Seoul 02841 (KR)
(72) Inventor: LEE, Young-Sun, Seoul 06317 (KR)
(74) Representative: Henderson, Helen Lee
(86) International application number: PCT/KR2021/004389
(87) International publication number: WO 2022/025387

(57) **Abstract**

The present invention relates to miRNA-4449, which is a biomarker that can be used to diagnose nonalcoholic steatohepatitis, and at least one miRNA selected from the group consisting of miRNA-15, miRNA-21, miRNA-29, miRNA-126, miRNA-151, miRNA-183, and miRNA-192. The prognosis of nonalcoholic steatohepatitis among nonalcoholic fatty liver diseases is poor unlike simple steatosis. According to the present invention, the level of the miRNA can be measured in the serum of a patient to provide an accurate diagnostic result of nonalcoholic steatohepatitis, thus making it possible to eliminate side effects caused by conventional invasive test methods, and enabling early diagnosis and appropriate treatment of steatohepatitis in a simple and economical manner.

## Description

### Technical Field

The following description relates to a biomarker composition and the like for diagnosing nonalcoholic steatohepatitis for non-invasive *in vitro* diagnosis, and more particularly, to a microRNA (miRNA) combination as a biomarker for identifying and diagnosing simple steatosis and steatohepatitis among nonalcoholic fatty liver diseases.

### Background Art

Nonalcoholic fatty liver disease (NAFLD) is a disease that can be diagnosed when alcohol, drugs, and other factors are excluded as the cause of such fatty liver in the case where fatty deposits are shown in liver tissue in a radiologic examination or a liver biopsy. In terms of a pathological mechanism, simple steatosis may progress to nonalcoholic fatty liver-associated cirrhosis via nonalcoholic steatohepatitis (NASH), but the nonalcoholic fatty liver disease is a term including a series of diseases. The prevalence of nonalcoholic fatty liver diseases in domestic studies has a great difference depending on a diagnosis method. The studies found that the prevalence of nonalcoholic fatty liver diseases when confirmed by a liver biopsy was 51.4%, and when diagnosed by an ultrasound, the prevalence was lower therethan at about 16 to 33%.

Since the liver biopsy is expensive, requires experts for interpretation, and has a risk of complications, in the 2018 American Liver Association guidelines, the liver biopsy is not recommended as a screening test for nonalcoholic fatty liver diseases, but recommended only when exclusion of other diseases is required when steatohepatitis or advanced liver fibrosis is suspected. However, a nonalcoholic fatty liver disease patient group has a higher overall mortality rate than a normal control group, and in particular, a cardiovascular disease mortality rate increases when the disease progresses to steatohepatitis. In health examinations of non-drinkers, since fatty liver diseases are observed on abdominal imaging tests and liver function is often abnormal on liver function tests, it is necessary to consider diagnosis and treatment with a possibility of nonalcoholic fatty liver diseases in mind.

The liver biopsy is still a standard diagnostic test for nonalcoholic fatty liver diseases because histological inflammation or fibrosis has a great influence on the prognosis. However, due to the high cost, invasiveness, and the risk of complications of the liver biopsy, it is unreasonable to be used in primary care. In addition, a small amount of liver tissue may not reflect the overall liver condition, and diagnostic concordance between pathologists may be low. Therefore, many efforts have been made to evaluate the histological severity of nonalcoholic fatty liver diseases through radiologic and biochemical examinations instead of the liver biopsies.

Accordingly, interest in non-invasive methods for diagnosis and severity classification of nonalcoholic fatty liver diseases is increasing, but there is still a lot of lack of studies using miRNAs. There have been attempts to analyze miRNA using conventional PCR and use the analyzed miRNA for diagnosis, but there is a limitation in that some of thousands of miRNAs are selected and analyzed through PCR. In addition, patents for the diagnosis of nonalcoholic steatohepatitis have been filed by domestic researchers, but there is a limit to the results of animal experiments.

Accordingly, the present inventors have intensively researched a non-invasive diagnostic method for distinguishing patients who have progressed to steatohepatitis among patients with nonalcoholic fatty liver diseases as a diagnostic method that is immediately applicable in the field, and then have completed the present invention.

### Disclosure of the Invention

### Technical Goals

An aspect provides miRNA-4449, which is a biomarker capable of identifying simple steatosis and steatohepatitis among nonalcoholic fatty liver diseases and further provides at least one miRNA selected from the group consisting of miRNA-15, miRNA-21, miRNA-29, miRNA-126, miRNA-151, miRNA-183, and miRNA-192.

Another aspect provides a composition and a kit capable of identifying and diagnosing simple steatosis and steatohepatitis among nonalcoholic fatty liver diseases.

Yet another aspect provides a pharmaceutical composition for preventing or treating nonalcoholic steatohepatitis including a substance for inhibiting the expression or activity of miRNA-4449 and a substance for inhibiting the expression or activity of at least one miRNA selected from the group consisting of miRNA-15, miRNA-21, miRNA-29, miRNA-126, miRNA-151, miRNA-183, and miRNA-192.

However, the technical aspects of the present invention are not limited to the aforementioned purpose and other aspects which are not mentioned may be clearly understood by those skilled in the art from the following description.

### Technical Solutions

According to an aspect, there is provided a biomarker for identifying and diagnosing simple steatosis and steatohepatitis among nonalcoholic fatty liver diseases including miRNA-4449 and further including at least one miRNA selected from the group consisting of miRNA-15, miRNA-21, miRNA-29, miRNA-126, miRNA-151, miRNA-183, and miRNA-192.

The biomarker may be preferably a combination of miRNA-4449, miRNA-21, miRNA-151, and miRNA-192, more preferably a combination of miRNA-4449, miRNA-15, miRNA-21, miRNA-29, miRNA-126, miRNA-151, miRNA-183, and miRNA-192.

According to another aspect, there is provided a composition for identifying and diagnosing simple steatosis and steatohepatitis among nonalcoholic fatty liver diseases including a preparation capable of measuring the expression level of the biomarker, that is, a preparation capable of measuring the expression level of miRNA-4449 and further including a preparation capable of measuring the expression level of at least one miRNA selected from the group consisting of miRNA-15, miRNA-21, miRNA-29, miRNA-126, miRNA-151, miRNA-183, and miRNA-192.

The composition for identifying and diagnosing of the present invention may include preferably a preparation capable of measuring the expression levels of miRNA-4449, miRNA-21, miRNA-151, and miRNA-192, more preferably a preparation capable of measuring the expression levels of miRNA-4449, miRNA-15, miRNA-21, miRNA-29, miRNA-126, miRNA-151, miRNA-183, and miRNA-192.

In an example embodiment of the present invention, the miRNA-4449 may include or consist of a nucleotide sequence represented by SEQ ID NO: 1.

In another example embodiment of the present invention, the miRNA-15 may be miRNA-15b-3p (SEQ ID NO: 2), the miRNA-21 may be miRNA-21-5p (SEQ ID NO: 3), the miRNA-29 may be miRNA-29b-3p (SEQ ID NO: 4), the miRNA-126 may be miRNA-126-5p (SEQ ID NO: 5), the miRNA-151 may be miRNA-151a-3p (SEQ ID NO: 6), the miRNA-183 may be miRNA-183-5p (SEQ ID NO: 7), and the miRNA-192 may be miRNA-192-5p (SEQ ID NO: 8).

**[Table 1]**

| **miRNA** | **miRNA sequence** | **SEQ ID NO** |
|---|---|---|
| miRNA-4449 | cgucccggggcugcgcgaggca | SEQ ID NO: 1 |
| miRNA-15b-3p | cgaaucauuauuugcugcucua | SEQ ID NO: 2 |
| miRNA-21-5p | uagcuuaucagacugauguuga | SEQ ID NO: 3 |
| miRNA-29b-3p | uagcaccauuugaaaucaguguu | SEQ ID NO: 4 |
| miRNA-126-5p | cauuauuacuuuugguacgcg | SEQ ID NO: 5 |
| miRNA-151a-3p | cuagacugaagcuccuugagg | SEQ ID NO: 6 |
| miRNA-183-5p | uauggcacugguagaauucacu | SEQ ID NO: 7 |
| miRNA-192-5p | cugaccuaugaauugacagcc | SEQ ID NO: 8 |

According to yet another aspect, there is provided a kit for identifying and diagnosing simple steatosis and steatohepatitis among nonalcoholic fatty liver diseases including a preparation capable of measuring the expression level of the miRNA.

In an example embodiment of the present invention, the preparation capable of measuring the expression level of the miRNA may be an oligonucleotide, a primer, or a probe having a sequence complementary to the miRNA.

According to still another aspect, there is provided an information providing method or a diagnosis method for identifying and diagnosing simple steatosis and steatohepatitis among nonalcoholic fatty liver patients including measuring the expression level of miRNA-4449 and the expression level of at least one miRNA selected from the group consisting of miRNA-15, miRNA-21, miRNA-29, miRNA-126, miRNA-151, miRNA-183, and miRNA-192 from biological samples of patients in need of identifying and diagnosing simple steatosis and nonalcoholic steatohepatitis.

In an example embodiment of the present invention, the patients may be patients diagnosed with nonalcoholic fatty liver diseases, and the biological sample may be at least one selected from the group consisting of blood, serum, and plasma.

In another example embodiment of the present invention, the method may further include determining as nonalcoholic steatohepatitis when the expression level of the measured miRNA is higher than that of the simple steatosis patient by comparing the measured miRNA expression level with the expression level of the miRNA corresponding to the measured miRNA in the simple steatosis patient after the measuring step, and the expression level of miRNA to be determined as the nonalcoholic steatohepatitis may be 1.5 times or higher, preferably 2 times or higher than the simple steatosis patient.

According to still yet another aspect, there is provided a pharmaceutical composition for preventing or treating nonalcoholic steatohepatitis including a preparation for inhibiting the expression of miRNA-4449 and further including a preparation for inhibiting the expression of at least one miRNA selected from the group consisting of miRNA-15, miRNA-21, miRNA-29, miRNA-126, miRNA-151, miRNA-183, and miRNA-192.

In an example embodiment of the present invention, the preparation may be at least one selected from the group consisting of siRNA, an aptamer, an antisense oligonucleotide, and a compound.

According to still yet another aspect, there are provided uses of miRNA-4449 and at least one miRNA selected from the group consisting of miRNA-15, miRNA-21, miRNA-29, miRNA-126, miRNA-151, miRNA-183, and miRNA-192 for preparing a drug for preventing or treating nonalcoholic steatohepatitis.

According to still yet another aspect, there is provided a method for preventing or treating nonalcoholic steatohepatitis including administering, to subject, a preparation for inhibiting the expression of miRNA-4449 and further administrating a preparation for inhibiting the expression of at least one miRNA selected from the group consisting of miRNA-15, miRNA-21, miRNA-29, miRNA-126, miRNA-151, miRNA-183, and miRNA-192.

According to still yet another aspect, there is provided a screening method of a drug for preventing or treating nonalcoholic steatohepatitis, including the following steps:
(1) treating liver cells with a candidate substance; (2) measuring the expression level of miRNA-4449 and the expression level of at least one miRNA selected from the group consisting of miRNA-15, miRNA-21, miRNA-29, miRNA-126, miRNA-151, miRNA-183, and miRNA-192 of the cells; and (3) selecting the candidate substance as a drug for preventing or treating nonalcoholic steatohepatitis when the miRNA expression levels measured in step (2) are lower than those before treatment with the candidate substance.

### Effects

The present invention relates to, miRNA-4449, which is a biomarker capable of diagnosing nonalcoholic steatohepatitis, and at least one miRNA selected from the group consisting of miRNA-15, miRNA-21, miRNA-29, miRNA-126, miRNA-151, miRNA-183, and miRNA-192. The prognosis of steatohepatitis among nonalcoholic fatty liver diseases is poor unlike simple steatosis. According to the present invention, the level of the miRNA can be measured in the serum of a patient to provide an accurate diagnostic result of nonalcoholic steatohepatitis, thereby making it possible to eliminate side effects caused by conventional invasive test methods, and enabling early diagnosis and appropriate treatment of steatohepatitis in a simple and economical manner.

### Brief Description of Drawings

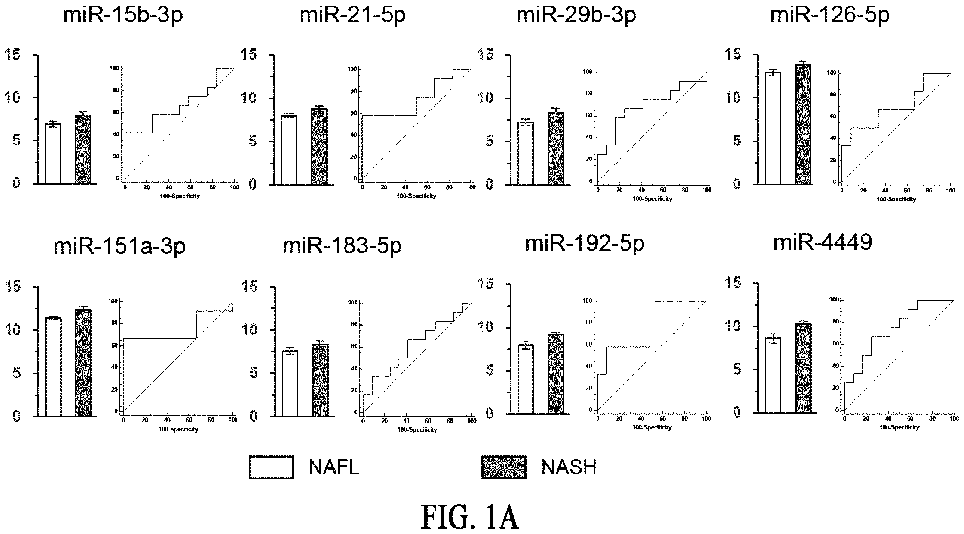

FIG. 1A illustrates a diagnostic ability of nonalcoholic steatohepatitis of each of eight miRNAs of which expression levels are increased in nonalcoholic steatohepatitis.

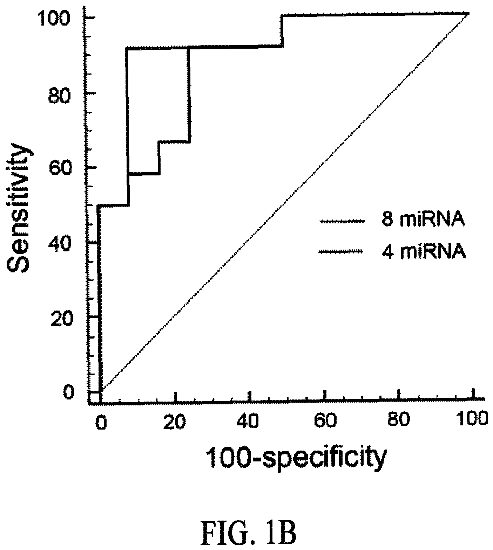

FIG. 1B illustrates a diagnostic ability of nonalcoholic steatohepatitis according to a combination of four miRNAs (miRNA-4449, miRNA-21, miRNA-151, and miRNA-192) and a combination of eight miRNAs (miRNA-4449, miRNA-15, miRNA-21, miRNA-29, miRNA-126, miRNA-151, miRNA-183, and miRNA-192).

### Best Mode for Carrying Out the Invention

The present inventors have made intensive research on a non-invasive test method for the diagnosis of nonalcoholic steatohepatitis capable of replacing a liver biopsy and as a result, have confirmed that miRNA-4449, miRNA-15, miRNA-21, miRNA-29, miRNA-126, miRNA-151, miRNA-183, and miRNA-192 are expressed in patients with nonalcoholic steatohepatitis compared to patients with simple steatosis, and then have completed the present invention.

Accordingly, the present invention provides a combination of miRNAs including, miRNA-4449, which is a biomarker capable of confirming the progression to steatohepatitis among patients with nonalcoholic fatty liver diseases and further including at least one miRNA selected from the group consisting of miRNA-15, miRNA-21, miRNA-29, miRNA-126, miRNA-151, miRNA-183, and miRNA-192.

Further, the present invention provides a composition for identifying and diagnosing simple steatosis and steatohepatitis among patients with nonalcoholic fatty liver diseases including a preparation capable of measuring the expression level of miRNA-4449 and further including a preparation capable of measuring the expression level of at least one miRNA selected from the group consisting of miRNA-15, miRNA-21, miRNA-29, miRNA-126, miRNA-151, miRNA-183, and miRNA-192 from biological samples of the patients.

In the present invention, the "diagnosis" means determining the susceptibility of a subject to a specific disease or disorder, determining whether a subject currently has a specific disease or disorder, determining the prognosis of a subject suffering from a specific disease or disorder, or therametrics (e.g., monitoring the conditions of a subject to provide information about treatment efficacy).

In the present invention, the "identifying" refers to diagnosing, judging, or discriminating a specific disease condition, and in particular, in the sense of which disease is accurately distinguished from diseases that share some clinically similar characteristics, the present invention used the term "identifying the disease". However, in terms of confirming a specific disease, the term may be used interchangeably with terms such as diagnosis, judgment, discrimination, and identification.

For the purpose of the present invention, the identifying in the present invention means differentiating between simple steatosis and nonalcoholic steatohepatitis among nonalcoholic fatty liver diseases. The simple steatosis refers to a simple benign disease in which triglycerides are accumulated in the liver, but the nonalcoholic steatohepatitis refers to a progressive liver disease characterized by accompanying inflammation or fibrosis with fatty liver. Since the prognosis and application of treatment regimens for both diseases are different, it is important to distinguish between simple steatosis and nonalcoholic steatohepatitis.

More preferably, the identifying in the present invention may mean confirming whether patients with nonalcoholic fatty liver diseases remain in a simple steatosis state or develop nonalcoholic steatohepatitis, that is, differentiating nonalcoholic steatohepatitis from simple steatosis.

The present invention is characterized by using a microRNA (miRNA) biomarker as a non-invasive tool to identify nonalcoholic steatohepatitis from simple steatosis.

More specifically, the present invention provides a technique of measuring the expression levels of miRNA-4449 and at least one miRNA selected from the group consisting of miRNA-15, miRNA-21, miRNA-29, miRNA-126, miRNA-151, miRNA-183, and miRNA-192 present in a sample to identify that a patient of the corresponding sample has nonalcoholic steatohepatitis when the miRNA is relatively overexpressed. In the present invention, the case where the miRNA is relatively overexpressed is based on the expression level of miRNA corresponding to the measured miRNA in a patient with simple steatosis, and in order to determine that the patient of the corresponding sample has nonalcoholic steatohepatitis, the miRNA expression level should be about 1.5 times higher than the miRNA expression level of the patient with simple steatosis, and preferably about 2 times or more overexpression should be confirmed.

In order to measure the expression level of miRNA-4449 and the expression of at least one miRNA selected from the group consisting of miRNA-15, miRNA-21, miRNA-29, miRNA-126, miRNA-151, miRNA-183, and miRNA-192 present in the sample, various preparations capable of detecting miRNA may be used.

Further, a kit for identifying and diagnosing simple steatosis and nonalcoholic steatohepatitis of the present invention may include a preparation capable of measuring the expression level of miRNA-4449 and further include a preparation capable of measuring the expression level of at least one miRNA selected from the group consisting of miRNA-15, miRNA-21, miRNA-29, miRNA-126, miRNA-151, miRNA-183, and miRNA-192 from a biological sample, and the preparation refers to a molecule that can be used to detect the biomarker of the present invention by confirming the expression level of miRNA.

In the present invention, an antisense oligonucleotide, a primer, a probe, and/or an antibody that specifically binds to miRNA may be used as the preparation capable of measuring the expression level of the miRNA, and the expression level of miRNA may be measured by performing known methods used for measuring a gene expression level, such as PCR using a primer or hybridization using a probe. In addition to the measurable preparation, the kit may include various tools and reagents known in the art for facilitating detection, for example, a suitable carrier, a label material capable of generating a detectable signal, a stabilizer, and the like.

In the present invention, the biological sample may be a non-invasive sample, and the non-invasive sample may be blood, serum, urine, or saliva, preferably blood or serum, more preferably serum, but is not limited thereto.

In the present invention, the miRNA is a concept including variants and mimics capable of having the same functional action as the miRNA nucleic acid molecule even though a functional equivalent of the oligonucleotide constituting the miRNA, for example, some nucleotide sequences of the miRNA oligonucleotide have been modified by deletion, substitution, or insertion.

In the present invention, the "antisense oligonucleotide" is a nucleotide sequence that binds complementarily to the miRNA to inhibit the expression, but is not limited thereto, and may include antisenseRNA and antagonist miRNA.

In the present invention, the "primer" refers to a short nucleic acid sequence capable of forming base pairs with a complementary template and serving as a starting point for copying a template strand, as a nucleic acid sequence having a short free hydroxyl group. The primer of the present invention may be chemically synthesized using methods known in the art, such as a phosphoramidite solid support method.

In the present invention, the "probe" refers to a nucleic acid fragment such as RNA or DNA consisting of several to hundreds of bases that may specifically bind to miRNA, which is labeled to confirm the presence or absence of a specific miRNA. The probe may be prepared in the form of an oligonucleotide probe, a single-stranded DNA probe, a double-stranded DNA probe, an RNA probe, or the like, and may be labeled with biotin, FITC, rhodamine, DIG, or the like, or labeled with a radioisotope or the like.

In the present invention, the kit may be a kit including essential elements required to perform RT-PCR, but is not limited thereto. In the case of an RT-PCR kit as an example, in addition to each primer pair specific to a marker gene, a test tube or other suitable containers, a reaction buffer, deoxynucleotides (dNTPs), Taq-polymerase and reverse transcriptase, DNase, an RNase inhibitor, DEPC-water, sterile water, and the like may be included.

Further, the present invention may provide an information providing method for identifying and diagnosing simple steatosis and steatohepatitis among nonalcoholic fatty liver diseases including measuring the expression level of miRNA-4449 and the expression level of at least one miRNA selected from the group consisting of miRNA-15, miRNA-21, miRNA-29, miRNA-126, miRNA-151, miRNA-183, and miRNA-192 in a biological sample of a patient. The information providing method may further include determining as steatohepatitis when the measured expression level of miRNA is higher than the expression level of a patient with simple steatosis.

The measuring of the expression level of miRNA may be performed by RT-PCR, competitive RT-PCR, real-time RT-PCR, quantitative RT-PCR, RNase protection assay (RPA), Northern blotting, DNA chips, DNA-silver nanoclusters (DNA/AgNC) sensors, or a kit method that directly binds to measure the expression level, but is not limited thereto.

In the present invention, the miRNA expression inhibitor is characterized by using a specific inhibitor to the miRNA, and the expression inhibitor may reduce the level of the miRNA in a cell by reducing the expression level of the miRNA or promoting the degradation thereof, or reduce the activity by specifically binding to the miRNA, and may be selected from the group consisting of siRNA, an aptamer, an antisense oligonucleotide and a compound. The compound may be used with any compound that inhibits the expression of miRNA among antioxidants, substances that affect cell growth, and cell signaling substances. However, the compound is not limited thereto, and may be used with any substance that inhibits the expression of the miRNA.

In the present invention, "siRNA" refers to a small RNA fragment having a size of 21 to 25 nucleotides produced by cleavage of double-stranded RNA by a dicer, which specifically binds to mRNA having a complementary sequence to inhibit the expression. For the purpose of the present invention, the "siRNA" may specifically bind to miRNA-4449 and at least one miRNA selected from the group consisting of miRNA-15, miRNA-21, miRNA-29, miRNA-126, miRNA-151, miRNA-183, and miRNA-192 to inhibit the expression of the miRNA. The siRNA may be chemically or enzymatically synthesized. The preparation method of siRNA is not particularly limited, and methods known in the art may be used. For example, the preparation method includes a method of directly chemically synthesizing siRNA, a synthesis method of siRNA using *in vitro* transcription, a method of cleaving long double-stranded RNA synthesized by *in vitro* transcription using an enzyme, an expression method through intracellular delivery of an shRNA expression plasmid or viral vector, an expression method through intracellular delivery of a polymerase chain reaction (PCR)-induced siRNA expression cassette, etc., but is not limited thereto.

In the present invention, the introduction of the miRNA inhibitor into cells may be performed using a microinjection method, a calcium phosphate co-precipitation method, an electroporation method, and a liposome method, but is not limited thereto.

In the present invention, the "prevention" means any action that delays inflammation and fibrosis of liver tissue or abnormalities thereof by administration of the composition according to the present invention. The "treatment" refers to all actions that improve or beneficially change inflammation and fibrosis of liver tissue and consequent metabolic abnormalities by administration of the pharmaceutical composition according to the present invention. In addition, a subject to be administered with the pharmaceutical composition for treating nonalcoholic steatohepatitis of the present invention is not limited as long as the subject is mammals, but may be preferably humans, more specifically patients with nonalcoholic fatty liver diseases.

In the present invention, the pharmaceutical composition may further include suitable carriers, excipients, and diluents commonly used in the preparation of the pharmaceutical composition.

In the present invention, the "carrier" is also called a vehicle, and refers to a compound that facilitates the addition of proteins or peptides into cells or tissues, and for example, dimethylsulfoxide (DMSO) is a commonly used carrier that facilitates the injection of many organic substances into the cells or tissues of living organisms.

In the present invention, the "diluent" is defined as a compound diluted in water that not only stabilizes a biologically active form of a target protein or peptide, but also dissolves the protein or peptide. Salts dissolved in a buffer solution are used as diluents in the art. A commonly used buffer solution is phosphate-buffered saline because the buffer solution imitates a salt state of a human solution. Since the buffer salt may control the pH of the solution at a low concentration, the buffer diluent rarely modifies the biological activity of the compound. Compounds containing azelaic acid used herein may be administered to human patients by themselves or as a pharmaceutical composition mixed with other ingredients or with suitable carriers or excipients, like combination therapy.

In addition, the pharmaceutical composition for preventing or treating nonalcoholic steatohepatitis of the present invention may be formulated and used in the form of external preparations, such as powders, granules, tablets, capsules, suspensions, emulsions, syrups, aerosols, etc., and sterile injectable solutions, according to general methods. The carrier, the excipient, and the diluent which may be included in the composition may include lactose, dextrose, sucrose, oligosaccharide, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. In the case of the preparation, the preparation may be prepared by using a diluent or an excipient, such as a filler, an extender, a binder, a wetting agent, a disintegrating agent, and a surfactant, which are commonly used. Solid preparations for oral administration include tablets, pills, powders, granules, capsules, and the like, and these solid preparations may be prepared by mixing at least one or more excipients, for example, starch, calcium carbonate, sucrose or lactose, gelatin, and the like. Further, lubricants such as magnesium stearate and talc may be used in addition to simple excipients. Liquid preparations for oral administration may correspond to suspensions, oral liquids, emulsions, syrups, and the like, and may include various excipients, for example, a wetting agent, a sweetener, an aromatic agent, a preserving agent, and the like, in addition to water and liquid paraffin which are commonly used as simple diluents. Preparations for parenteral administration include a sterile aqueous solution, a non-aqueous solution, a suspension, an emulsion, a lyophilizing agent, and a suppository. As the non-aqueous solution and the suspension, propylene glycol, polyethylene glycol, vegetable oil such as olive oil, injectable ester such as ethyl oleate, and the like may be used. As a base compound of the suppository, witepsol, macrogol, tween 61, cacao butter, laurinum, glycerogelatin, and the like may be used.

The pharmaceutical composition of the present invention may be administered orally or parenterally, preferably parenterally, and in the case of parenteral administration, the pharmaceutical composition may be administered through intramuscular injection, intravenous injection, subcutaneous injection, intraperitoneal injection, topical administration, transdermal administration, etc.

A suitable dose of the pharmaceutical composition of the present invention may be variously prescribed by factors, such as a formulation method, an administration type, age, weight, and sex of a patient, a pathological condition, food, an administration time, an administration route, an excretion rate, and response susceptibility.

The pharmaceutical composition of the present invention may be formulated by using a pharmacologically acceptable carrier and/or excipient according to a method that may be easily performed by those skilled in the art to be prepared in a unit dosage form or prepared by introduction into a multi-dosage container. In this case, the formulation may also be the form of solutions, suspensions, or emulsions in oily or aqueous media or the form of extracts, powders, granules, tablets, or capsules, and may additionally include a dispersant or a stabilizer.

The present invention may have various modifications and various example embodiments and specific example embodiments will be illustrated in the drawings and described in detail in the detailed description. However, this does not intend to limit the present invention within specific example embodiments, and it should be understood that the present invention covers all the modifications, equivalents, and replacements within the idea and technical scope of the present invention. In the interest of clarity, not all details of the relevant art are described in detail in the present specification in so much as such details are not necessary to obtain a complete understanding of the present invention.

### Mode for Carrying Out the Invention

### [Experimental method]

### - Analysis of diagnostic ability of miRNA on nonalcoholic steatohepatitis

Serums were collected from 24 patients diagnosed with nonalcoholic fatty liver diseases through histological examination and analyzed. Among the 24 patients, 12 patients had simple steatosis and 12 patients had nonalcoholic steatohepatitis. RNA was isolated from the serums collected from these patients and the expression of miRNAs was analyzed. Expression analysis of miRNAs was conducted through RNA sequencing, and the results are shown in FIG. 1 as a ROC curve.

### [Experiment Results]

As a result of comparing miRNA expression in a simple steatosis group and a nonalcoholic steatohepatitis group, there were a total of 38 miRNAs showed statistical significance with a difference of 2 times or more in both groups. In a nonalcoholic steatohepatitis group, 26 miRNAs were significantly increased and 12 miRNAs were significantly decreased. Among the miRNAs increased in nonalcoholic steatohepatitis, 8 miRNAs with sufficient expression levels were selected, which were miR-15b-3p, miR-21-5p, miR-29b-3p, miR-126-5p, miR-151a-3p, miR-183-5p, miR-192-5p, and miRNA-4449. Table 2 shows the diagnostic ability (AUROC), statistical significance (p-value), sensitivity, and specificity for nonalcoholic steatohepatitis of each miRNA. Among them, miRNAs having the significant AUROC were four types of miR-21-5p, miR-151a-3p, miR-192-5p, and miR-4449 having a significant difference between the two groups.

**[Table 2]**

| miRNA | Simple steatosis (n = 12) | Steatohepatitis (n = 12) | AUROC | p-value | Sensitivity | Specificity |
|---|---|---|---|---|---|---|
| miR-15b-3p | 6.96 ± 0.33 | 7.90 ± 0.45 | 0.667 (0.447-0.844) | 0.154 | 41.67 | 100 |
| miR-21-5p | 8.01 ± 0.22 | 8.79 ± 0.33 | 0.736 (0.518-0.893) | 0.032 | 58.33 | 100 |
| miR-29b-3p | 7.24 ± 0.38 | 8.33 ± 0.52 | 0.694 (0.475-0.864) | 0.089 | 58.33 | 83.33 |
| miR-126-5p | 12.93 ± 0.33 | 13.84 ± 0.39 | 0.694 (0.475-0.864) | 0.084 | 50.00 | 91.67 |
| miR-151a-3p | 11.39 ± 0.16 | 12.37 ± 0.34 | 0.750 (0.533-0.902) | 0.030 | 66.67 | 100 |
| miR-183-5p | 7.57 ± 0.39 | 8.29 ± 0.49 | 0.618 (0.399-0.807) | 0.322 | 66.67 | 58.33 |
| miR-192-5p | 7.97 ± 0.45 | 9.15 ± 0.27 | 0.771 (0.555-0.916) | 0.007 | 100 | 50.00 |
| miR-4449 | 8.64 ± 0.58 | 10.28 ± 0.33 | 0.743 (0.525-0.898) | 0.018 | 66.67 | 75.00 |
| Combine of 8 miRNAs | | | 0.924 (0.739-0.992) | <0.001 | 91.67 | 91.67 |
| Combine of 4 miRNAs | | | 0.875 (0.676-0.973) | <0.001 | 91.67 | 75.00 |

On the other hand, when the 8 miRNAs were combined, the AUROC value for the diagnosis of nonalcoholic steatohepatitis was 0.924, and when 4 miRNAs of miR-21-5p, miR-151a-3p, miR-192-5p, and miR-4449 were combined, the AUROC value for the diagnosis of nonalcoholic steatohepatitis was 0.875, and as a result, there was no significant difference between the two groups.

From the results, it can be seen that in the serum of patients with nonalcoholic fatty liver diseases, the expressions of miR-15b-3p, miR-21-5p, miR-29b-3p, miR-126-5p, miR-151a-3p, miR-183-5p, miR-192-5p, and miRNA-4449 are analyzed, and these miRNAs are combined to diagnose nonalcoholic steatohepatitis and the miRNAs can be used as a useful biomarker capable of distinguishing simple steatosis patients from steatohepatitis patients among patients with nonalcoholic fatty liver diseases.

As described above, specific parts of the present invention have been described in detail, and it will be apparent to those skilled in the art that these specific techniques are merely preferred example embodiments, and the scope of the present invention is not limited thereto. Therefore, the substantial scope of the present invention will be defined by the appended claims and their equivalents.

### Industrial Applicability

The present invention relates to miRNA-4449, which is a biomarker capable of diagnosing nonalcoholic steatohepatitis, and at least one miRNA selected from the group consisting of miRNA-15, miRNA-21, miRNA-29, miRNA-126, miRNA-151, miRNA-183, and miRNA-192. Since it is possible to accurately determine and diagnose nonalcoholic steatohepatitis with the poor prognosis unlike simple steatosis by measuring the level of the miRNA in the patient's serum, the present invention can be usefully used in the field of prevention, treatment and diagnosis of nonalcoholic steatohepatitis.

## Claims

1. A biomarker composition for identifying and diagnosing simple steatosis and steatohepatitis among nonalcoholic fatty liver diseases comprising miRNA-4449 and
further comprising at least one selected from the group consisting of miRNA-15, miRNA-21, miRNA-29, miRNA-126, miRNA-151, miRNA-183, and miRNA-192.

2. The biomarker composition of claim 1, wherein the miRNA-4449 consists of a nucleotide sequence represented by SEQ ID NO: 1.

3. A composition for identifying simple steatosis and steatohepatitis among nonalcoholic fatty liver diseases comprising a preparation capable of measuring a expression level of miRNA-4449 and
further comprising a preparation capable of measuring the expression level of at least one miRNA selected from the group consisting of miRNA-15, miRNA-21, miRNA-29, miRNA-126, miRNA-151, miRNA-183, and miRNA-192.

4. The composition of claim 3, wherein the preparation capable of measuring the miRNA expression level is an oligonucleotide, a primer, or a probe having a sequence complementary to the miRNA.

5. A kit for identifying simple steatosis and steatohepatitis among nonalcoholic fatty liver diseases comprising the composition of claim 3.

6. An information providing method for identifying and diagnosing simple steatosis and nonalcoholic steatohepatitis comprising:
measuring expression levels of miRNA-4449 and at least one miRNA selected from the group consisting of miRNA-15, miRNA-21, miRNA-29, miRNA-126, miRNA-151, miRNA-183, and miRNA-192 from a biological sample of a patient in need of identifying and diagnosing the simple steatosis and the nonalcoholic steatohepatitis.

7. The information providing method of claim 6, wherein the patient is a patient diagnosed with nonalcoholic fatty liver diseases, and
the biological sample is at least one selected from the group consisting of blood, serum, and plasma.

8. The information providing method of claim 6, further comprising:
comparing the measured miRNA expression level with the expression level of the miRNA corresponding to the measured miRNA in the simple steatosis patients after the measuring step.

9. The information providing method of claim 8, further comprising:
determining as the nonalcoholic steatohepatitis when the expression level of each miRNA of patients in need of identification and diagnosis is 1.5 times higher than that of the simple steatosis patients after the comparing step.

10. A diagnosis method for identifying and diagnosing simple steatosis and nonalcoholic steatohepatitis comprising:
measuring expression levels of miRNA-4449 and at least one miRNA selected from the group consisting of miRNA-15, miRNA-21, miRNA-29, miRNA-126, miRNA-151, miRNA-183, and miRNA-192 from a biological sample of a patient in need of identifying and diagnosing the simple steatosis and the nonalcoholic steatohepatitis.

11. The diagnosis method of claim 10, comprising:
comparing the measured miRNA expression level with the expression level of the miRNA corresponding to the measured miRNA in the simple steatosis patients after the measuring step.

12. A pharmaceutical composition for preventing or treating nonalcoholic steatohepatitis comprising a preparation for inhibiting an expression of miRNA-4449 and
further comprising a preparation for inhibiting the expression of at least one miRNA selected from the group consisting of miRNA-15, miRNA-21, miRNA-29, miRNA-126, miRNA-151, miRNA-183, and miRNA-192.

13. The pharmaceutical composition of claim 12, wherein the preparation is at least one selected from the group consisting of siRNA, an aptamer, an antisense oligonucleotide, and a compound.

14. A method for preventing or treating nonalcoholic steatohepatitis comprising:
administering, to a subject, a preparation for inhibiting an expression of miRNA-4449 and
further administering a preparation for inhibiting the expression of at least one miRNA selected from the group consisting of miRNA-15, miRNA-21, miRNA-29, miRNA-126, miRNA-151, miRNA-183, and miRNA-192.

15. A screening method of a drug for preventing or treating nonalcoholic steatohepatitis, comprising following steps:
(1) treating liver cells with a candidate substance;
(2) measuring an expression level of miRNA-4449 and the expression level of at least one miRNA selected from the group consisting of miRNA-15, miRNA-21, miRNA-29, miRNA-126, miRNA-151, miRNA-183, and miRNA-192 of the cells; and
(3) selecting the candidate substance as a drug for preventing or treating the nonalcoholic steatohepatitis when the miRNA expression levels measured in step (2) are lower than those before treatment with the candidate substance.
